# EUROPEAN PATENT APPLICATION

(11) **EP 4 834 274 A1**
(43) Date of publication of application: **04.11.2026**
(21) Application number: 24913474.3
(22) Date of filing: 27.11.2024
(51) Int. Cl.: A61C 8/00, B23K 26/352, A61L 27/06

(54) **DENTAL IMPLANT SURFACE TREATMENT METHOD AND DENTAL IMPLANT SURFACE-TREATED USING SAME**

(30) Priority: 29.12.2023 KR 20230196360
(71) Applicant: Osstemimplant Co., Ltd., Gangseo-gu Seoul 07789 (KR)
(72) Inventor: RHO, Keon Ho, Seoul 07789 (KR); KIM, Kyung Il, Seoul 07789 (KR); PARK, Jae Jun, Seoul 07789 (KR)
(74) Representative: Germain Maureau
(86) International application number: PCT/KR2024/019029
(87) International publication number: WO 2025/143581

(57) **Abstract**

A method for treating a surface of a dental implant to have an osseointegration topography on the surface and a dental implant surface-treated by the method are presented. The method for treating a surface of a dental implant according to the present invention comprises preparing an implant body having a screw thread formed on an outer portion thereof and made of pure titanium or made of a titanium alloy material composed of titanium and one or more selected from aluminum (Al), silicon (Si), vanadium (V), niobium (Nb), zirconium (Zr), molybdenum (Mo), chromium (Cr), tin (Sn), tantalum (Ta), palladium (Pd), and combinations thereof; a primary surface treatment step of primarily treating the outer surface of the implant body having the outer screw thread formed thereon by sandblasting or laser surface treatment; and a secondary surface treatment step of secondarily treating the surface of the implant body subjected to the primary surface treatment by using an etching solution, wherein in the secondary surface treatment step, any one selected from hydrochloric acid, sulfuric acid, phosphoric acid, nitric acid, hydrofluoric acid, and hydrogen peroxide, or a mixture in which two or more selected from hydrochloric acid, sulfuric acid, phosphoric acid, nitric acid, hydrofluoric acid, and hydrogen peroxide are mixed, is used as the etching solution.

## Description

### [Technical Field]

The present invention relates to a method for treating a surface of a dental implant, and more particularly, to a method for treating a surface of a dental implant having an osseointegration topography on the surface and a dental implant surface-treated by the method.

### [Background Art]

An implant originally means a substitute for restoring human tissue when the human tissue is lost, but in dentistry, it means an artificially made tooth. Such an implant procedure is widely used as an alternative procedure capable of restoring or reconstructing the original function of a patient's own tooth when the tooth is lost or the tooth function is degraded.

Generally, a dental implant is composed of pure titanium, and in order to promote fast and strong osseointegration, a surface thereof is preferably roughened by an SLA (Sandblast with Large grit and Acid etched) technology. For reference, the SLA surface treatment technology is an implant surface treatment technology including a sandblasting treatment for implementing a macro surface shape by evenly spraying an irregular ceramic powder such as Al₂O₃, TiO₂, Zr₂O₃, or Hydroxyapatite at a high pressure onto a surface of a machined implant body, and an etching treatment for implementing a micro surface shape through an acid treatment.

Pure titanium hardly causes an immune rejection response in the human body due to its high biocompatibility. However, if the mechanical strength of the material is low, a problem may occur. More specifically, due to insufficient mechanical strength (such as tensile strength or fatigue strength), fracture of the implant is likely to occur during the procedure, and for this reason, there is a disadvantage in that the probability of clinical failure is high.

As an alternative to this, a technology of using a titanium alloy having high mechanical strength while being suitable for a living body by mixing another metal with titanium as a dental implant material has been proposed. For example, WO 1997-029624 describes utilizing a titanium-zirconium (Ti-Zr) alloy including more than 5% by weight and less than 25% by weight of zirconium (Zr) based on 100% by weight in total as an implant material.

The titanium-zirconium (Ti-Zr) alloy disclosed in WO 1997-029624 has better mechanical properties than a material obtained by cold-working pure titanium. According to WO 1997-029624, the alloy may be manufactured by sequentially performing hot forging and subsequent cold working. Here, the forging process may be performed at a temperature of 850 °C or higher, or alternatively, within an alpha/beta phase transition range of 770 to 830 °C.

The success or failure of a dental implant procedure depends on how fast and strongly the implant interacts with surrounding bone tissue. From the viewpoint of fast and strong osseointegration, it is preferable to implement the surface topography of the titanium-zirconium implant like the surface of the existing pure titanium implant surface-treated by SLA.

However, since the titanium-zirconium alloy has a different microstructure from pure titanium, it is not easy to implement its surface like the surface of the pure titanium implant. That is, there is a problem that it is difficult to implement the surface topography of the titanium-zirconium implant like the surface topography of the pure titanium implant under the same surface treatment conditions as those for the surface treatment of the pure titanium implant.

In addition, SLA surface treatment is generally performed as a surface treatment method for an implant composed of pure titanium and a titanium alloy, but the SLA surface treatment may cause a problem in that osseointegration characteristics are not sufficiently implemented due to an irregular shape provided to the surface. When an implant in which osseointegration characteristics are not sufficiently implemented is implanted in the human body, perfect osseointegration may not be formed between the implant and bone, which may excessively increase a procedure period or lead to a procedure failure.

As described above, various surface treatment methods have been proposed as a method for improving osseointegration characteristics of conventional implants, and research and development of a surface treatment method for further improving osseointegration characteristics are required.

### [Prior Art Document]

(Patent Document 0001) International Patent Publication WO 1997-029624 (published on June 10, 1997)

### [Detailed Description of the Invention]

### [Technical Problem]

A technical problem to be solved by the present invention is to provide a method for treating a surface of a dental implant capable of implementing a surface topography equivalent to or more regular and uniform than SLA surface treatment of a pure titanium implant on the surface thereof while using pure titanium and a titanium alloy having improved mechanical strength as materials, and a dental implant surface-treated by the method.

### [Means for Solving the Problem]

According to one embodiment as a means for solving the problem, there is provided a method for treating a surface of a dental implant to have an osseointegration topography on the surface, the method comprising: preparing a titanium-zirconium (Ti-Zr) implant body having a screw thread formed on an outer portion thereof; a primary surface treatment step of implementing a macro surface shape on the outer surface of the implant body having the outer screw thread formed thereon through a sandblasting treatment; and a secondary surface treatment step of implementing a micro surface on the surface of the implant body subjected to the primary surface treatment by using an etching solution, wherein surface treatment conditions in the secondary surface treatment step are such that an etching time is 5 minutes to 10 minutes, an etching temperature is 90 to 120 °C, and any one selected from hydrochloric acid, sulfuric acid, phosphoric acid, nitric acid, hydrofluoric acid, and hydrogen peroxide, or a mixture in which two or more selected from hydrochloric acid, sulfuric acid, phosphoric acid, nitric acid, hydrofluoric acid, and hydrogen peroxide are mixed, is used as the etching solution.

In one embodiment, the titanium-zirconium (Ti-Zr) implant body may be preferably a titanium-zirconium alloy comprising 14 to 16 parts by weight of zirconium (Zr) and 84 to 86 parts by weight of titanium (Ti) based on 100 parts by weight in total.

According to another embodiment as a means for solving the problem, there is provided a method for treating a surface of a dental implant to have an osseointegration topography on the surface, the method comprising: preparing an implant body having a screw thread formed on an outer portion thereof and made of pure titanium or made of a titanium alloy material composed of titanium and one or more selected from aluminum (Al), silicon (Si), vanadium (V), niobium (Nb), zirconium (Zr), molybdenum (Mo), chromium (Cr), tin (Sn), tantalum (Ta), palladium (Pd), and combinations thereof; a primary surface treatment step of forming grid-pattern grooves on the outer surface of the implant body having the outer screw thread formed thereon by using a laser; and a secondary surface treatment step of secondarily treating the surface of the implant body subjected to the primary surface treatment by using an etching solution, wherein surface treatment conditions in the secondary surface treatment step are such that an etching time is 4 minutes to 60 minutes, an etching temperature is 70 to 120 °C, and any one selected from hydrochloric acid, sulfuric acid, phosphoric acid, nitric acid, hydrofluoric acid, and hydrogen peroxide, or a mixture in which two or more selected from hydrochloric acid, sulfuric acid, phosphoric acid, nitric acid, hydrofluoric acid, and hydrogen peroxide are mixed, is used as the etching solution.

And in another embodiment, the primary surface treatment step may comprise: a primary laser treatment process of processing a plurality of first grooves continuous in a first direction and spaced apart in a second direction; and a secondary laser treatment process of processing a plurality of second grooves continuous in the second direction and spaced apart in the first direction to form a crater at each intersection of the first grooves and the second grooves.

Here, it is preferable that the craters formed on the outer surface of the implant body by the primary laser treatment process and the secondary laser treatment process have a higher crater density in an outer lower screw thread portion than in an outer upper screw thread portion.

According to another embodiment as a means for solving the problem, there is provided a dental implant implanted into an alveolar bone to form an artificial tooth root, wherein an internal groove into which an abutment for supporting a prosthesis is coupled is formed to a specific depth from a top surface thereof, a screw thread for implantation into the alveolar bone is formed on an outer surface thereof, and the outer surface having the screw thread formed thereon is surface-treated by the surface treatment method according to the one embodiment and another embodiment to have an osseointegration topography on the outer surface.

### [Advantageous Effects]

According to the method for treating a surface of a dental implant according to an embodiment of the present invention, while using pure titanium or a titanium alloy having superior mechanical properties compared to pure titanium as a material, a topography on the surface thereof may be implemented in an equivalent shape or more uniformly as compared to a surface topography of a pure titanium implant, so that a dental implant having stable osseointegration characteristics can be provided.

### [Brief Description of Drawings]

FIG. 1 is a process schematic diagram for explaining a method for treating a surface of a dental implant according to one embodiment of the present invention.
FIGS. 2 and 3 are SEM images showing a surface state of a Ti-15Zr implant body primarily surface-treated through sandblasting after a secondary surface treatment (etching treatment).
FIG. 4 is a process schematic diagram for explaining a method for treating a surface of a dental implant according to another embodiment of the present invention.
FIG. 5 is a schematic view illustrating a laser surface treatment process performed in a primary surface treatment step in another embodiment of the present invention.
FIG. 6 shows an SEM image and a cross-sectional schematic view showing an implant surface state that has undergone a primary surface treatment step in another embodiment of the present invention.
FIG. 7 is a graph comparing surface roughness of an implant body surface after surface treatment only with a laser and after surface treatment through hydrochloric acid + sulfuric acid etching (this embodiment) after laser surface treatment.
FIG. 8 is a front view of a dental implant according to another embodiment of the present invention.

### [Mode for Carrying out the Invention]

Hereinafter, preferred embodiments of the present invention will be described in detail with reference to the accompanying drawings.

Terms used in the specification are merely used to describe specific embodiments, and are not intended to limit the present invention. Singular expressions include plural expressions unless the context clearly indicates otherwise.

In the present specification, terms such as "comprise" or "have" should be understood as designating that features, numbers, steps, operations, components, parts, or a combination thereof described in the specification exist, and do not preclude the existence or addition possibility of one or more other features, numbers, steps, operations, components, parts, or combinations thereof in advance.

In addition, terms such as first and second may be used to describe various components, but the components should not be limited by the terms. The terms are used only for the purpose of distinguishing one component from another component.

In the description with reference to the accompanying drawings, the same reference numerals are assigned to the same components, and overlapping descriptions thereof will be omitted. In describing the present invention, when it is determined that a detailed description of related known technologies may unnecessarily obscure the subject matter of the present invention, the detailed description will be omitted.

The present invention relates to a method for treating a surface of a dental implant to have an osseointegration topography on the surface, where the 'dental implant' means a part that is implanted into an alveolar bone of an implant target region and serves as a pillar, that is, serves as an artificial tooth root, and after implantation, osseointegrates with the corresponding alveolar bone to generate a strong coupling force.

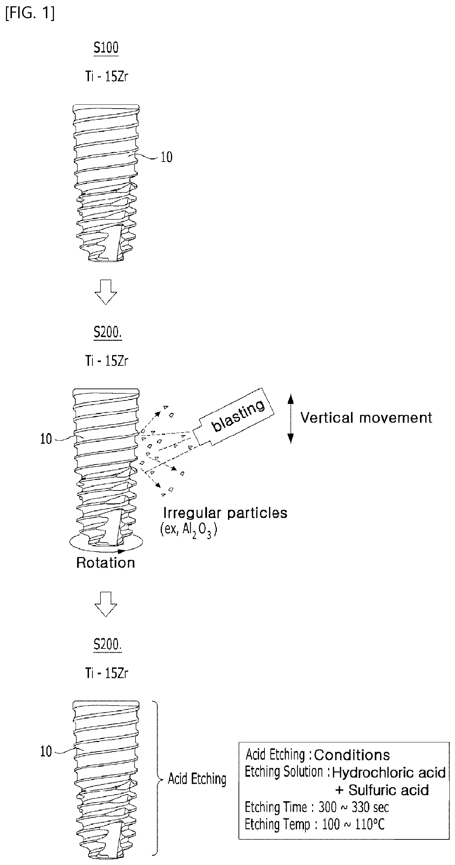

FIG. 1 is a process schematic diagram for explaining a method for treating a surface of a dental implant according to one embodiment of the present invention.

Referring to FIG. 1, a surface treatment method according to one embodiment of the present invention comprises a step of preparing an implant body (S100), a primary surface treatment step of implementing a macro surface shape on a surface of the implant body 100 by sandblasting (S200), and a secondary surface treatment step of implementing a micro surface on the surface of the implant body subjected to the primary surface treatment by using an etching solution (S300).

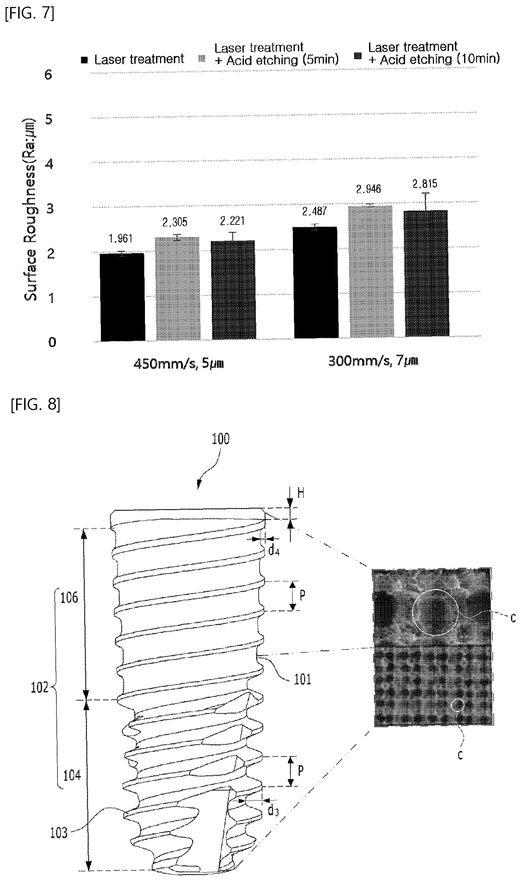

In the step of preparing the implant body (S100), the implant body 100 is prepared through machining. Here, the implant body refers to a shape in which an internal groove, into which an abutment supporting a prosthesis is coupled, is formed to a specific depth from a top surface, and a screw thread coupled to a bone of an implantation target portion by a screw fastening method is formed on an outer surface thereof, and preferably may be a shape illustrated in FIG. 8 later.

In the step of preparing the implant body (S100), the implant body 100 may be made of an alloy made by mixing titanium (Ti) and zirconium (Zr) at a specific ratio, that is, a titanium-zirconium (Ti-Zr) alloy as a material. The implant body may be preferably made of a titanium-zirconium alloy comprising 14 to 16 parts by weight of zirconium (Zr) and 84 to 86 parts by weight of titanium (Ti) based on 100 parts by weight in total.

When less than 14 parts by weight of zirconium is mixed with respect to 100 parts by weight in total, mechanical strength (tensile strength and fatigue strength) is not significantly different from pure titanium, and when the amount of zirconium exceeds 16 parts by weight with respect to 100 parts by weight in total, mechanical strength is significantly improved compared to pure titanium, but surface treatment in a subsequent process becomes difficult, and biocompatibility decreases, thereby significantly increasing the possibility of occurrence of procedural side effects.

In the primary surface treatment step (S200), the outer surface of the implant body 100 is primarily surface-treated through sandblasting. In the primary surface treatment step (S100), preferably, an irregular ceramic powder such as Al₂O₃, TiO₂, Zr₂O₃, or Hydroxyapatite is evenly sprayed at a high pressure onto the machined surface of the implant body to roughen the surface. Here, as a blasting condition, a blasting pressure may be at least 1.5 bar.

In the secondary surface treatment step (S200), a predetermined etching treatment is performed on the surface of the implant body 100 by using an acidic etching solution. In addition to the macro surface shape implemented on the surface of the implant body 100 by sandblasting, a micro surface shape may be additionally implemented by such secondary surface treatment.

Surface treatment conditions in the secondary surface treatment step (S200) of secondarily treating the surface of the implant body subjected to the primary surface treatment by using an etching solution are such that an etching time is 5 minutes to 10 minutes, an etching temperature is 90 to 120 °C, and any one selected from hydrochloric acid, sulfuric acid, phosphoric acid, nitric acid, hydrofluoric acid, and hydrogen peroxide, or a mixture in which two or more selected from hydrochloric acid, sulfuric acid, phosphoric acid, nitric acid, hydrofluoric acid, and hydrogen peroxide are mixed may be used as the etching solution.

Preferably, surface treatment conditions in the secondary surface treatment step are such that an etching time is 300 seconds (5 minutes), an etching temperature is 110 °C, and a mixture in which hydrochloric acid and sulfuric acid are mixed may be used as an etching solution.

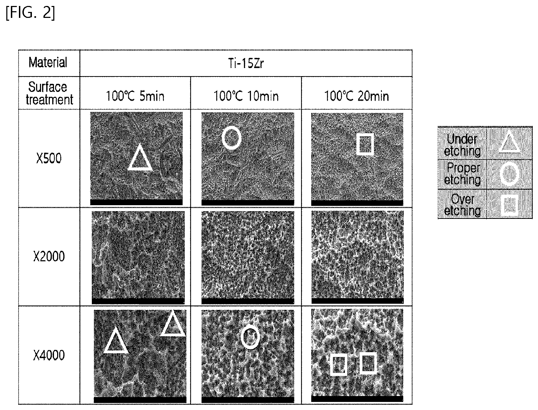

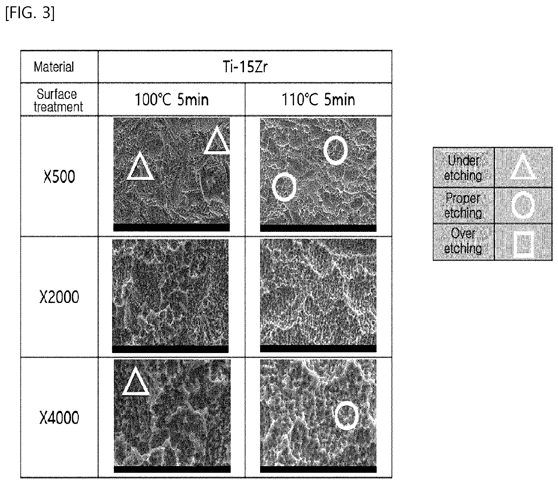

FIGS. 2 and 3 are SEM images showing surface states of a Ti-15Zr (an alloy composed of 15 parts by weight of zirconium based on 100 parts by weight in total and the remainder being titanium) implant body primarily surface-treated through sandblasting after a secondary surface treatment (etching treatment), and are SEM images showing surface states when surface treatment is performed with an etching solution composed of hydrochloric acid + sulfuric acid while varying an etching time and a temperature.

First, referring to FIG. 2, looking at the results of performing the surface treatment while changing the etching time to 5 minutes, 10 minutes, and 20 minutes while maintaining the etching temperature at 100 °C in the same manner, when the etching time was 5 minutes, the degree of roughness of the surface was not entirely uniform, and when the etching time was 20 minutes, the degree of roughness of the surface was good compared to the result of performing for 5 minutes, but it can be seen that etching was excessively performed compared to the result of performing for 10 minutes.

And, looking at the surface treatment results (FIG. 3) when only the etching temperature was changed while keeping the etching time the same at 5 minutes, it can be seen that the degree of roughness was entirely uniformly formed under an etching temperature condition of 110 °C compared to an etching temperature condition of 100 °C. In particular, it can be seen that the surface treatment result under these conditions (etching time 5 minutes, etching temperature 110 °C) is similar to the surface treatment result at an etching time of 10 minutes and an etching temperature of 100 °C (see FIG. 2).

According to these test results, in performing the secondary surface treatment (etching treatment) using the etching solution composed of hydrochloric acid + sulfuric acid, as the treatment conditions, when the etching temperature is 100 °C, the etching time is preferably 10 minutes, and when the etching temperature is 110 °C, the etching time is preferably 5 minutes.

However, considering the effect of the etching time on the mass productivity of the product (if the etching time is long, the mass productivity of the product may decrease accordingly), most preferably, the etching treatment is performed for 5 minutes at an etching temperature of 110 °C.

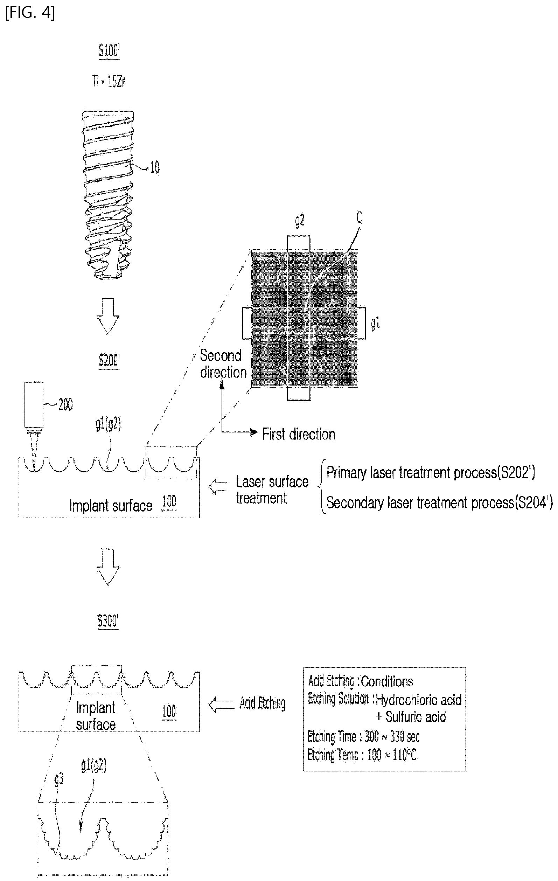

FIG. 4 is a process schematic diagram for explaining a method for treating a surface of a dental implant according to another embodiment of the present invention.

Referring to FIG. 4, a surface treatment method according to another embodiment of the present invention comprises a step of preparing an implant body (S100'), a step of primarily treating the surface of the implant body with a laser to provide a macro surface shape (S200'), and a step of secondarily treating the surface with an etching solution to provide a micro surface shape (S300').

In the step of preparing the implant body (S100'), the implant body 100 is prepared through machining. Here, the implant body is a form in which an internal groove, into which an abutment supporting a prosthesis is coupled, is formed to a specific depth from a top surface, a form integrated with the abutment, a form in which an abutment supporting a prosthesis is coupled above the top surface, and the like, and preferably may be a form illustrated in FIG. 8 later.

In the step of preparing the implant body (S100'), the implant body 100 may be an alloy made by mixing titanium (Ti) and zirconium (Zr) at a specific ratio, that is, a titanium-zirconium (Ti-Zr) alloy as a material. The implant body 100 may be preferably made of a titanium-zirconium alloy comprising 14 to 16 parts by weight of zirconium (Zr) and 84 to 86 parts by weight of titanium (Ti) based on 100 parts by weight in total.

When less than 14 parts by weight of zirconium is mixed with respect to 100 parts by weight in total, mechanical strength (tensile strength and fatigue strength) is not significantly different from pure titanium, and when the amount of zirconium exceeds 16 parts by weight with respect to 100 parts by weight in total, mechanical strength is significantly improved compared to pure titanium, but surface treatment in a subsequent process becomes difficult, and biocompatibility decreases, thereby significantly increasing the possibility of occurrence of procedural side effects.

In the surface treatment method according to another embodiment of the present invention, the implant body 100 in the step of preparing the implant body (S100') may be pure titanium or a titanium alloy material composed of titanium and one or more selected from aluminum (Al), silicon (Si), vanadium (V), niobium (Nb), zirconium (Zr), molybdenum (Mo), chromium (Cr), tin (Sn), tantalum (Ta), palladium (Pd), and combinations thereof.

More preferably, the implant body 100 may be pure titanium, a titanium-aluminum-vanadium (Ti-Al-V)-based alloy, or a titanium-zirconium (Ti-Zr)-based alloy.

In the primary surface treatment step (S200'), the outer surface of the implant body 100 is primarily surface-treated using a laser. Preferably, an operation of forming regular pattern grooves using a laser may be performed on all or a part of the implant surface. Here, the regular pattern may be a grid pattern in which two groups of grooves arranged in different directions cross each other at various angles including right angles.

In this embodiment, specifically, the primary surface treatment step (S200') may be composed of a primary laser treatment process (S202') of processing a plurality of first grooves (hereinafter, referred to as a 'first groove group') continuous in a first direction and spaced apart in a second direction, and a secondary laser treatment process (S204') of processing a plurality of second grooves (hereinafter, referred to as a 'second groove group') continuous in the second direction and spaced apart in the first direction to form a crater c at each intersection of the first grooves g1 and the second grooves g2.

In the present embodiment, the first direction may be a circumferential direction of the implant body, and the second direction may be a longitudinal direction of the implant body orthogonal to the first direction. Of course, the present invention is not limited thereto, and the first direction may be the same as or opposite to a direction in which a screw thread formed on an implant surface is formed, for example, and the second direction may be a direction diagonally inclined with respect to the first direction.

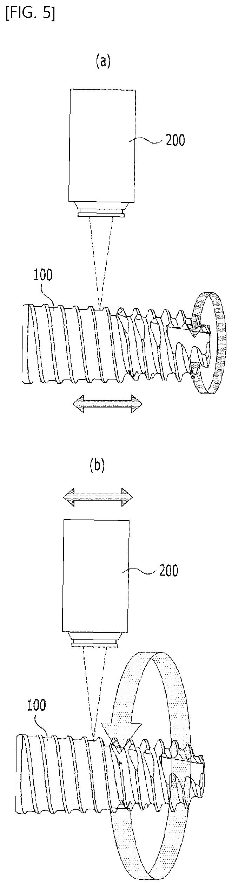

In performing the primary surface treatment using a laser, a grid-patterned groove may be formed by rotating and moving forward and backward the rotating device in a direction of an arrow with respect to a laser generator 200 (equipment irradiating a laser at a set intensity), which is a relatively fixed body, as shown in the surface treatment schematic diagram of FIG. 5(a), in a state in which the implant body 100, which is a surface treatment target, is inserted into a rotating device (not shown).

On the contrary, as shown in the surface treatment schematic diagram of FIG. 5(b), in a state where the implant 100 to be surface-treated is fixed to a fixing device (not shown), a grid-patterned groove may be formed by moving the laser generator 200 (equipment irradiating a laser at a set intensity), which is a relatively movable body, in a rotation and an arrow direction with respect to the implant 100 fixed to the fixing device.

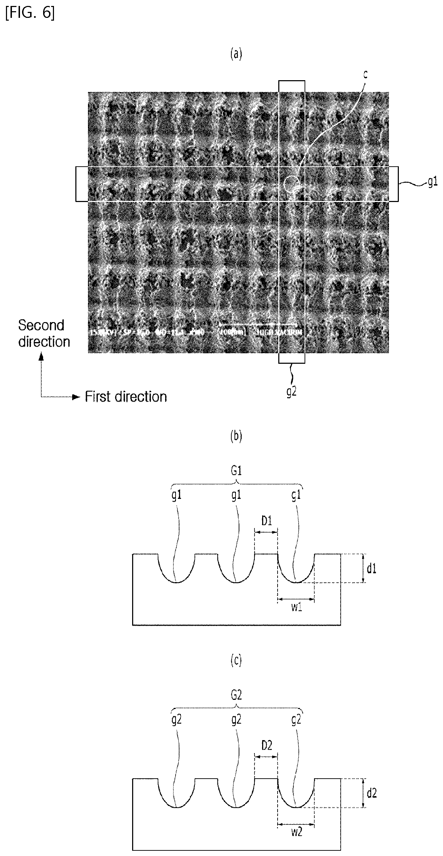

FIG. 6 is an SEM image and a cross-sectional schematic view showing an implant surface state that has undergone a primary surface treatment step. Preferably, a first groove g1 and a second groove g2 may be formed in a grid pattern orthogonal to each other. At this time, a width w1 of the first groove g1 and a width w2 of the second groove g2 are identical (w1=w2), and a depth d1 of the first groove g1 and a depth d2 of the second groove g2 may also be formed at the same depth (d1=d2).

Here, it is preferable to understand that the width w1 of the first groove g1 and the width w2 of the second groove g2 mean an average width of grooves g1 of a first groove group G1 and grooves g2 of a second groove group G2 formed in plural, and it is preferable to understand that the depth d1 of the first groove g1 and the depth d2 of the second groove g2 also mean an average depth of the grooves g1 and g2 included in the corresponding group.

The widths w1 and w2 of the first groove g1 and the second groove g2 may preferably be 1 to 100 µm. This is because if the widths w1 and w2 of the first groove g1 and the second groove g2 formed in directions crossing each other are less than 1 µm, the surface area increasing effect is insignificant, and osseointegration characteristics may be deteriorated, and if the widths of the first groove g1 and the second groove g2 exceed 100 µm, the durability of the implant body may be deteriorated.

The depths d1 and d2 of the first groove g1 and the second groove g2 may preferably be 1 to 100 µm. If the depths d1 and d2 of the first groove g1 and the second groove g2 are less than 1 µm, the surface area increasing effect is insignificant, and if the depths of the first groove g1 and the second groove g2 exceed 100 µm, uniformity of a micro groove g3 may drop when forming the micro groove g3 on surfaces of the first groove g1 and the second groove g2 through the secondary surface treatment step thereafter.

In the present embodiment, a distance D1 between one first groove g1 and another neighboring first groove g1 is preferably 1 to 100 µm in consideration of a density of the craters c per unit area and an osseointegration characteristic according thereto, and a distance D2 between one second groove g2 formed in a direction crossing the first groove g1 and another neighboring second groove g2 is also preferably 1 to 100 µm.

In the secondary surface treatment step (S300'), a predetermined etching treatment is performed on the surface of the implant body by using an acidic etching solution. Due to such secondary surface treatment, a micro-sized fine groove is formed on the surface of each groove g1, g2 formed on the surface of the implant body by primary laser processing (see FIG. 4), so that the entire surface area can be greatly increased.

As surface treatment conditions in the secondary surface treatment step (S300') using an etching solution, an etching time is 4 minutes to 60 minutes, an etching temperature is 70 to 120 °C, and any one selected from hydrochloric acid, sulfuric acid, phosphoric acid, nitric acid, hydrofluoric acid, and hydrogen peroxide, or a mixture in which two or more selected from hydrochloric acid, sulfuric acid, phosphoric acid, nitric acid, hydrofluoric acid, and hydrogen peroxide are mixed may be used as the etching solution.

More preferably, the etching time is 7 minutes to 13 minutes, the etching temperature is 100 °C, and a hydrochloric acid + sulfuric acid solution is preferably used as an etching solution.

FIG. 7 is a graph comparing surface roughness of an implant body surface after surface treatment only with a laser and after proceeding with laser treatment as a primary surface treatment and hydrochloric acid + sulfuric acid etching surface treatment as a secondary surface treatment (this embodiment). Compared to when only laser treatment was performed, when surface treatment was performed using a hydrochloric acid + sulfuric acid etching method after laser treatment, the surface roughness significantly increased, and it can be seen that the surface roughness rather increased when the etching time was 5 minutes compared to when the etching time was 10 minutes.

For reference, among values shown in FIG. 7, a value having a unit of mm/s is a value representing a laser processing speed, and a value having a unit of µm is a value representing a depth of a groove (corresponding to d1 and d2 in FIG. 6) formed through laser processing.

Under a condition that the sizes are the same, the osseointegration characteristic combined with bone during the implant procedure is improved as the surface area of the dental implant increases, and the surface area increases as the surface roughness increases. In that respect, the graph of FIG. 7 may be interpreted as supporting that the surface treatment method according to this embodiment (hydrochloric acid + sulfuric acid etching after laser treatment) has a clear effect in terms of improving osseointegration performance.

According to the method for treating a surface of a dental implant according to the embodiment of the present invention described above, while using a titanium alloy (titanium-zirconium) having mechanical properties superior to those of pure titanium as a material, the topography on the surface thereof can be implemented to be equivalent to or greater than the surface topography of a pure titanium implant, so that a dental implant having stable osseointegration characteristics can be provided.

FIG. 8 is a front view of a dental implant surface-treated by the above-described surface treatment method.

Referring to FIG. 8, a dental implant 100 according to another aspect of the present invention is a structure implanted into an alveolar bone to form an artificial tooth root, and may include an outer surface 101 on which an external screw thread 103 is formed for implantation into the alveolar bone.

Here, the outer surface 101 having the outer screw thread 103 formed thereon may be surface-treated through a primary laser treatment process of processing a plurality of first grooves continuous in a first direction and spaced apart in a second direction, and a secondary laser treatment process of processing a plurality of second grooves continuous in the second direction and spaced apart in the first direction to form a crater at each intersection of the first grooves and the second grooves.

The implant 100 according to this embodiment may be made of a titanium alloy in which zirconium (Zr) occupies 14 to 16 parts by weight based on 100 parts by weight in total and the remainder is titanium (Ti), and the outer surface 101 is surface-treated by the above-described surface treatment method, thereby having an osseointegration topography capable of promoting fast and strong osseointegration with a bone (alveolar bone).

In particular, when a technique of primarily surface-treating with a laser and secondarily surface-treating with an etching solution mixed with hydrochloric acid and sulfuric acid among the above-described surface treatment methods is used, in forming a crater (a recessed groove formed at an intersection point as the first groove and the second groove intersect in FIG. 6 above) through laser processing, it is preferable that an outer lower screw thread section 104 has a higher crater density than an outer upper screw thread section 106 (see main enlarged image of FIG. 8).

As described above, when the crater density of the outer lower screw thread section 104 is increased compared to the outer upper screw thread section 106, that is, when the outer lower screw thread section is configured to have a rougher surface than the outer upper screw thread section, the effect of inhibiting bacterial proliferation and the osseointegration characteristic can be improved in a more balanced manner.

In the detailed description of the present invention above, only specific embodiments thereof have been described. However, it should be understood that the present invention is not limited to the specific forms mentioned in the detailed description, but rather includes all modifications, equivalents, and substitutes falling within the spirit and scope of the present invention defined by the appended claims.

### [Description of Reference Numerals]

100 : Implant
101 : Outer surface
103 : Outer screw thread
104 : Outer lower screw thread section
106 : Outer upper screw thread section
g1 : First groove
g2 : Second groove
c : Crater

## Claims

1. A method for treating a surface of a dental implant to have an osseointegration topography on the surface, the method comprising:
preparing an implant body having a screw thread formed on an outer portion thereof and made of pure titanium or a titanium-zirconium (Ti-Zr) alloy;
a primary surface treatment step of primarily treating the outer surface of the implant body having the outer screw thread formed thereon by sandblasting; and
a secondary surface treatment step of secondarily treating the surface of the implant body subjected to the primary surface treatment by using an etching solution,
wherein surface treatment conditions in the secondary surface treatment step are such that an etching time is 5 minutes to 10 minutes, an etching temperature is 90 to 120 °C, and any one selected from hydrochloric acid, sulfuric acid, phosphoric acid, nitric acid, hydrofluoric acid, and hydrogen peroxide, or a mixture in which two or more selected from hydrochloric acid, sulfuric acid, phosphoric acid, nitric acid, hydrofluoric acid, and hydrogen peroxide are mixed, is used as the etching solution.

2. The method according to claim 1, wherein the titanium-zirconium (Ti-Zr) implant body is a titanium-zirconium alloy comprising 14 to 16 parts by weight of zirconium (Zr) and 84 to 86 parts by weight of titanium (Ti) based on 100 parts by weight in total.

3. A method for treating a surface of a dental implant to have an osseointegration topography on the surface, the method comprising:
preparing an implant body having a screw thread formed on an outer portion thereof and made of pure titanium or made of a titanium alloy material composed of titanium and one or more selected from aluminum (Al), silicon (Si), vanadium (V), niobium (Nb), zirconium (Zr), molybdenum (Mo), chromium (Cr), tin (Sn), tantalum (Ta), palladium (Pd), and combinations thereof;
a primary surface treatment step of forming grid-pattern grooves on the outer surface of the implant body having the outer screw thread formed thereon by using a laser; and
a secondary surface treatment step of secondarily treating the surface of the implant body subjected to the primary surface treatment by using an etching solution,
wherein surface treatment conditions in the secondary surface treatment step are such that an etching time is 4 minutes to 60 minutes, an etching temperature is 70 to 120 °C, and any one selected from hydrochloric acid, sulfuric acid, phosphoric acid, nitric acid, hydrofluoric acid, and hydrogen peroxide, or a mixture in which two or more selected from hydrochloric acid, sulfuric acid, phosphoric acid, nitric acid, hydrofluoric acid, and hydrogen peroxide are mixed, is used as the etching solution.

4. The method according to claim 3, wherein the titanium-zirconium (Ti-Zr) implant body is a titanium-zirconium alloy comprising 14 to 16 parts by weight of zirconium (Zr) and 84 to 86 parts by weight of titanium (Ti) based on 100 parts by weight in total.

5. A method for treating a surface of a dental implant to have an osseointegration topography on the surface, the method comprising:
preparing an implant body made of pure titanium or made of a titanium alloy material composed of titanium and one or more selected from aluminum (Al), silicon (Si), vanadium (V), niobium (Nb), zirconium (Zr), molybdenum (Mo), chromium (Cr), tin (Sn), tantalum (Ta), palladium (Pd), and combinations thereof;
a primary surface treatment step of forming grid-pattern grooves on the outer surface of the implant body having the outer screw thread formed thereon by using a laser; and
a secondary surface treatment step of secondarily treating the surface of the implant body subjected to the primary surface treatment by using an etching solution,
wherein the primary surface treatment step comprises:
a primary laser treatment process of processing a plurality of first grooves continuous in a first direction and spaced apart in a second direction; and
a secondary laser treatment process of processing a plurality of second grooves continuous in the second direction and spaced apart in the first direction to form a crater at each intersection of the first grooves and the second grooves.

6. The method according to claim 5, wherein a density of the craters formed on the outer surface of the implant body by the primary laser treatment process and the secondary laser treatment process is higher in an outer lower screw thread portion than in an outer upper screw thread portion.

7. A dental implant implanted into an alveolar bone to form an artificial tooth root, wherein an internal groove into which an abutment for supporting a prosthesis is coupled is formed to a specific depth from a top surface thereof, a screw thread for implantation into the alveolar bone is formed on an outer surface thereof, and the outer surface having the screw thread formed thereon is surface-treated by the surface treatment method according to any one of claims 1 to 6 to have an osseointegration topography on the outer surface.
